(19) **Europäisches Patentamt**

**European Patent Office**

**Office européen des brevets**

(11) **EP 0 400 065 B1**

(12) **EUROPEAN PATENT SPECIFICATION**

(45) Date of publication and mention
of the grant of the patent:
**27.08.1997 Bulletin 1997/35**

(51) Int Cl.6: **C07K 7/23, A61K 38/09**

(86) International application number:
**PCT/US89/00528**

(21) Application number: **89902885.6**

(22) Date of filing: **09.02.1989**

(87) International publication number:
**WO 89/07450 (24.08.1989 Gazette 1989/20)**

(54) **LHRH ANALOG**

LHRH-ANALOG

ANALOGUE DE L'HORMONE DE LIBERATION DE L'HORMONE DE LUTEINISATION (LHRH)

(84) Designated Contracting States:
**BE CH DE ES FR GB GR IT LI NL SE**

(30) Priority: **10.02.1988 US 154681**

(43) Date of publication of application:
**05.12.1990 Bulletin 1990/49**

(73) Proprietor: **TAP Pharmaceuticals Inc.**
**Deerfield Illinois 60015 (US)**

(72) Inventors:
 • **HAVIV, Fortuna**
   **Deerfield, IL 60015 (US)**
 • **GREER, Jonathan**
   **Chicago, IL 60645 (US)**

(74) Representative: **Modiano, Guido, Dr.-Ing. et al**
**Modiano, Josif, Pisanty & Staub,**
**Baaderstrasse 3**
**80469 München (DE)**

(56) References cited:
| | |
|---|---|
| EP-A- 0 021 234 | EP-A- 0 257 742 |
| EP-A- 0 328 089 | FR-A- 2 307 543 |
| GB-A- 2 009 182 | US-A- 4 444 759 |
| US-A- 4 504 414 | US-A- 4 581 169 |
| US-A- 4 632 979 | US-A- 4 642 332 |
| US-A- 4 689 396 | |

 • **NESTOR, Journal of Andrology, 8 (1), S4-S8**
   **published January/February 1987**
 • **CREIGHTON, Proteins, W.H. Freeman and**
   **Company, New York, 47, published 1984**

Remarks:
Consolidated with 89102208.9/0328090 (European
application No./publication No.) by decision dated
26.02.92.

**Description**

Technical Field

The present invention relates to a novel "pseudo" decapeptide analog of LHRH wherein the nitrogen atom of one of the amide bonds is alkylated. The invention also relates to processes for preparing such compound and to pharmaceutical compositions containing such compound for use in modulating levels of sex hormones in male or female mammals.

Background Art

Luteinizing Hormone Releasing Hormone, known as LHRH or GnRH, is a decapeptide with the following formula: (pyro)Glu-His-Trp-Ser-Tyr-Gly-Leu-Arg-Pro-Gly-NH$_2$ LHRH is released from the hypothalamus and binds to a receptor on the pituitary gland, causing the release of LH (Luteinizing Hormone) and FSH (Follicle - Stimulating Hormone). Subsequently, LH and FSH act on the gonads to stimulate the synthesis of steroid sex hormones. The pulsatile release of LHRH, and thereby the release of LH and FSH, controls the reproductive cycle in domestic animals and humans. Acute doses of LHRH agonists increase the levels of LH and steroid sex hormones in both animals and humans. Paradoxically, chronic doses of these agonists suppress the levels of LH and steroid hormones. Consequently, the effect of multiple doses of LHRH agonists is to suppress estrogen formation in the female and suppress testosterone formation in the male. The same effect is observed in both animals and humans after administration of acute or chronic doses of LHRH antagonists.LHRH agonists are currently used or under clinical investigation for the treatment of several hormone dependent diseases such as prostate cancer, prostatic hypertrophy, endometriosis, uterine fibroids, precocious puberty and breast cancer. They have also been used as contraceptives. For a review of LHRH analogs see J. Sandow, et al. in "Hypothalamic Hormones. Chemistry, Physiology, and Clinical Applications", edited by D. Gupta and W. Voeters, p. 307 (1978).

Many biologically active LHRH analogs have been studied in animals and humans. All of them are effective by either intravenous, subcutaneous, or depot administration. Intranasal and intravaginal administrations are effective only at very high doses. All of the reported LHRH analogs show 0.1% to 1% potency following oral administration when compared to intraveneous doses. One of the major reasons for this low potency is that these peptides are degraded in the stomach by various proteolytic enzymes before reaching the blood system. It would be desirable to prepare analogs of LHRH that are stable against proteolytic enzymes and are biologically potent after oral administration in animals and humans.

Of interest as background to the present invention are the following documents:

GB-A-2 009 182 which discloses peptides which are effective in inhibiting the release of LH.

J.J. Nestor, Jr. et al, J. Med. Chem., vol. 25 (1982), pages 795-801 discloses some hydrophobic agonist analogs of LHRH.

M.J. Karten et al, Endocrine Review, vol. 7, no. 1 (1986), pages 44-66 discusses structure-function studies in relationship to the development of LHRH agonists and antagonists.

EP-A-0 021 234 discloses nonapeptide and decapeptide analogs of LHRH.

J.J. Nestor, Jr., J. Androl., vol. 8(1), 1987, pages S-4 to S-8 discusses the design of LHRH drug candidates.

EP-A-0 257 742 discloses a method of solid-phase synthesis of a polypeptide having a non-peptide bond and gives an example of the synthesis of an LHRH antagonist.

US-A-4444759 discloses peptide antagonists of LHRH.

S. Thaisrivongs et al., J. Med. Chem., vol. 29 (1986), pages 2088-2093 disclose the design and synthesis of a renin inhibitor which showed a prolonged duration of action in vivo.

FR-A-2307543 discloses nonapeptide and decapeptide LHRH analogs.

US-A-4234571 also discloses nonapeptide and decapeptide LHRH analogs.

Summary of the Invention

The present invention relates to a novel "pseudo" decapeptide derivative of LHRH. More particularly the present invention relates to a derivative of LHRH wherein the nitrogen atom of one of the amide bonds is alkylated.

Disclosure of the Invention

The compound of the present invention is N-Ac-D-2-Nal-D-4-Cl-Phe-D-3-Pal-Ser-N-Me-Tyr-D-Lys(N-epsilon-nicotinoyl)-Leu-Lys(N-epsilon-isopropyl)-Pro-D-AlaNH$_2$; or a pharmaceutically acceptable salt thereof.

This compound exhibits affinity for LHRH receptors.

For convenience in describing this invention and the reference examples, the conventional abbreviations for the various common amino acids are used as generally accepted in the peptide art as recommended by the IUPAC-IUB Commission on Biochemical Nomenclature, Biochemistry II, 1726 (1972). These represent L-amino acids, with the exception of any unnatural or natural amino acids which are otherwise designated as D-. All peptide sequences mentioned herein are written according to the generally accepted convention whereby the N-terminal amino acid is on the left and the C-terminal amino acid is on the right.

Other abbreviations which are useful in describing the invention are the following:

| Amino acids, protecting groups, reagents | Abbreviation |
| --- | --- |
| L-N-(epsilon)-isopropyllysyl | (isp)Lys |
| Arginine | Arg |
| t-Butoxycarbonyl | Boc |
| Benzyl | Bzl |
| Benzyloxycarbonyl | Cbz |
| N,N'-Dicyclohexylcarbodiimide | DCC |
| Histidine | His |
| 1-Hydroxybenzotriazole | HOBt |
| Leucine | Leu |
| Benzyl ester | OBzl |
| Phenylalanine | Phe |
| Proline | Pro |
| Pyroglutamic acid | (pyro)Glu |
| Serine | Ser |
| Tosyl | Tos |
| Tryptophan | Trp |
| Tyrosine | Tyr |
| N,N'-di-isopropylcarbodiimide | DIC |
| Alanine | Ala |
| L-N-methylserine | N-Me-Ser |
| 3-(pyridyl)-L-alanyl | 3-Pal |
| L-N-methyl-O-benzylseryl | N-Me-Ser(OBzl) |
| 3-(2-naphthyl)-D-alanyl | D-(2)-Nal |
| 4-Dimethylaminopyridine | DMAP |
| Benzotriazol-1-yloxy-tris(dimethyl-amino)phosphonium hexafluorophosphate | BOP |
| Bis(2-oxo-3-oxazolidinyl)phosphine chloride | BOPCl |

The sequence of LHRH has been shown to be

$$(pyro)Glu-His-Trp-Ser-Tyr-Gly-Leu-Arg-Pro-Gly-NH_2 .$$
$$\quad 1 \quad\ 2 \quad\ 3 \quad\ 4 \quad\ 5 \quad\ 6 \quad\ 7 \quad\ 8 \quad\ 9 \quad 10$$

The decapeptide of the invention in which the amino acid residues at particular places in the sequence have been replaced by other amino acid residues or other moieties can be abbreviated by showing the nature of the substitution, superscribed by the location, followed by LHRH as the parent.

Thus the sequence of the compound of the invention can be represented by:

$$[N-Ac-D-2-Nal-4-Cl-D-Phe^2-D-3-Pal-N-Me-Tyr^5-D-Lys^6-$$
$$(N-epsilon-nicotinoyl)-Lys^8-(N-epsilon-isopropyl)-D-$$
$$Ala^{10}]LHRH;$$

As used herein, the term "pharmaceutically acceptable salts" refers to salts that retain the desired biological activity of the parent compound and do not impart any undesired toxicological effects. Examples of such salts are (a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, naphthalenesulfonic acids, naphthalenedisulfonic acids, polygalacturonic acid; (b) salts with polyvalent metal cations such as zinc, calcium, bismuth, barium, magnesium, aluminum, copper, cobalt, nickel, cadmium, and the like; or with an organic cation formed from N,N'-dibenzylethylene-diamine or ethylenediamine; or (c) combinations, of (a) and (b), e.g., a zinc tannate salt and the like.

Effect and Utilities of the LHRH Antagonist

The LHRH antagonist compound of the invention is useful for treatment of precocious puberty, prostate cancer, prostatic hypertrophy, endometriosis, uterine fibroids, breast cancer, acne, premenstrual syndrome, polycystic ovary syndrome and diseases which result from excessive gonadal hormone production in either sex. The LHRH antagonist of the invention is also useful for controlling reproduction in both females and males.

In practice, an effective amount of the compound of the invention or a pharmaceutical composition containing the same is administered to the subject in need of, or desiring, such treatment. This compound or composition may be administered by any of a variety of routes depending upon the specific end use, including orally, parenterally (including subcutaneous, intramuscular and intraveneous administration), vaginally (particularly for contraception), rectally, buccally (including sublingually), transdermally or intranasally. The most suitable route in any given case will depend upon the use, particular active ingredient, the subject involved, and the judgment of the medical practitioner. The compound or composition may also be administered by means of slow-release, depot or implant formulations as described more fully herein below.

In general, to modulate levels of sex hormones in male or female mammals for the uses herein above described, it is expedient to administer the active ingredient in amounts between about 0.01 and 10 mg/kg body weight per day, preferably between about 0.1 and 5.0 mg/kg body weight per day. This administration may be accomplished by a single daily administration, by distribution over several applications or by slow release in order to achieve the most effective results.

The exact dose and regimen for administration of these compounds and compositions will necessarily be dependent upon the needs of the individual subject being treated, the type of treatment, the degree of affliction or need and, of course, the judgment of the medical practitioner. In general, parenteral administration requires lower dosage than other methods of administration which are more dependent upon absorption.

A further aspect of the present invention relates to pharmaceutical compositions containing as active ingredient the compound of the present invention which compositions comprise such compound in admixture with a pharmaceutically acceptable, non-toxic carrier. As mentioned above, such compositions may be prepared for use for parenteral (subcutaneous, intramuscular or intraveneous) administration, particularly in the form of liquid solutions or suspensions; for use in vaginal or rectal administration, particularly in semisolid forms such as creams and suppositories; for oral or buccal administration, particularly in the form of tablets or capsules, or intranasally, particularly in the form of powders, nasal drops or aerosols.

The compositions may conveniently be administered in unit dosage form and may be prepared by any of the methods well-known in the pharmaceutical art, for example as described in <u>Remington's Pharmaceutical Sciences,</u> Mack Publishing Company, Easton, PA., 1970. Formulations for parenteral administration may contain as common excipients sterile water or saline, polyalkylene glycols such as polyethylene glycol, oils of vegetable origin, hydrogenated naphthalens and the like. Formulations for inhalation administratiom may be solid and contain as excipients, for example, lactose, or may be aqueous or oily solutions for administration in the form of nasal drops. For buccal administration typical excipients include sugars, calcium stearate, magnesium stearate, pregelatinated starch, and the like.

It is particularly desirable to deliver the compound of the present invention to the subject over prolonged periods of time, for example, for periods of one week to one year from a single administration. Various slow release, depot or implant dosage forms may be utilized. For example, a dosage form may contain a pharmaceutically acceptable non-toxic salt of the compound of the invention which has a low degree of solubility in body fluids, for example, (a) an acid addition salt with a polybasic acid such as phosphoric acid, sulfuric acid, citric acid, tartaric acid, tannic acid, pamoic acid, alginic acid, polyglutamic acid, naphthalene mono- or di-sul£onic acids, polygalacturonic acid, and the like; (b) a salt with a polyvalent metal cation such as zinc, calcium, bismuth, barium, magnesium, aluminum, copper, cobalt, nickel, cadmium and the like, or with an organic cation formed from e.g., N,N'-dibenzylethylenediamine or ethylenediamine; or (c) combinations of (a) and (b) e.g. a zinc tannate salt. Additionally, the compound of the present invention or, preferably, a relatively insoluble salt such as those just described, may be formulated in a gel, for example, an aluminum monostearate gel with, e.g. sesame oil, suitable for injection. Particularly preferred salts are zinc salts, zinc tannate salts, pamoate salts, and the like. Another type of slow release depot formulation for injection would contain the compound or salt dispersed or encapsulated in a slow degrading, non-toxic, non-antigenic polymer such as a polylactic acid/polyglycolic acid polymer for example as described in U.S. Patent No. 3,773,919. The compound of the invention or, preferably, relatively insoluble salts such as those described above may also be formulated in cholesterol matrix pellets, particularly for use in animals. Additional slow release, depot or implant formulations, e.g. liposomes, are well known in the literature. See, for example, <u>Sustained and Controlled Release Drug Delivery Systems,</u> J.R. Robinson ed., Marcel Dekker, Inc., New York, 1978. Particular reference with respect to LHRH type compounds may be found, for example, in U.S. Patent No. 4,010,125.

<u>Synthesis of the Peptide</u>

The polypeptide of the present invention may be synthesized by any techniques that are known to those skilled in the art. For solid phase peptide synthesis, a summary of the many techniques may be found in J.M. Stewart and J.D. Young, <u>Solid Phase Peptide Synthesis,</u> W.H. Freeman Co., San Francisco, 1963 and J. Meienhofer, <u>Hormonal Proteins</u> and <u>Peptides</u>, Vol. 2., p.46, Academic Press (New York), 1973. For classical solution synthesis see G. Schroder and K. Lupke, <u>The Peptides,</u> vol. 1, Academic Pres (New York), 1965.

In general, these methods comprise the sequential addition of one or more amino acids or suitably protected amino acids to a growing peptide chain. Normally, either the amino or carboxyl group of the first amino acid is protected by a suitable protecting group. The protected or derivatized amino acid can then be either attached to an inert solid support or utilized in solution by adding the next amino acid in the sequence having the complimentary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently, to afford the final polypeptide. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide.

A particularly preferred method of preparing the compound of the present invention involves solid phase peptide synthesis.

In this particularly preferred method the alpha-amino function of the amino acids is protected by an acid or base sensitive group. Such protecting groups should have the properties of being stable to the conditions of peptide linkage formation, while being readily removable without destruction of the growing peptide chain or racemization of any of the chiral centers contained therein. Suitable protecting groups are <u>t</u>-butyloxycarbonyl (Boc), benzyloxycarbonyl (Cbz), biphenylisopropyloxycarbonyl, <u>t</u>-amyloxycarbonyl, isobornyloxycarbonyl, (alpha,alpha)-dimethyl-3,5-dimethoxybenzyloxycarbonyl, <u>o</u>-nitrophenylsulfenyl, 2-cyano-<u>t</u>-butyloxycarbonyl, 9-fluorenylmethyloxycarbonyl and the like. The <u>t</u>-butyloxycarbonyl (Boc) protecting group is preferred.

Particularly preferred side chain protecting groups are, for tyrosine: benzyl, <u>o</u>-bromobenzyloxycarbonyl, 2,6-dichlorobenzyl, isopropyl, cyclohexyl, cyclopentyl and acetyl; for serine: benzyl and tetrahydropyranyl.

In the solid phase peptide synthesis method, the C-terminal amino acid is attached to a suitable solid support. Suitable solid supports useful for the above synthesis are those materials which are inert to the reagents and reaction conditions of the stepwise condensation-deprotection reactions, as well as being insoluble in the media used. Suitable solid supports are chloromethylpolystyrene-divinylbenzene polymer, hydroxymethyl-polystyrene-divinylbenzene polymer, and the like. Chloromethyl-polystyrene-1% divinylbenzene polymer is especially preferred. The coupling to the chloromethyl polystyrene-divinylbenzene type of resin is made by means of the reaction of the alpha-N-protected amino acid, especially the Boc-amino acid, as its cesium, tetramethylammonium, triethylammonium, 1,5-diazabicyclo-[5.4.0] undec-5-ene, or similar salt. The coupling reaction is accomplished in a solvent such as ethanol, acetonitrile, N,N-dimethylformamide (DMF), and the like, with the chloromethyl resin at an elevated temperature, for example between about 40° and 60°C, for from about 12 to 48 hours. Preferred reagents and reaction conditions involve the coupling of an alpha-N-Boc amino acid cesium salt with the resin in DMF at about 50°C for about 24 hours. The alpha-N-Boc-amino acid is attached to the benzhydrylamine resin by means of N,N'-dicyclohexylcarbodiimide (DCC) or N,N'-diisopropylcarbodiimide (DIC) with or without 1-hydroxybenzotriazole (HOBT), benzotriazol-1-yloxy-tris-(dimethylamino) phosphonium-hexafluorophosphate (BOP) or bis(2-oxo-3-oxazolidinyl)phosphine chloride (BOPCl), mediated coupling for from about 1 to about 24 hours, preferably about 12 hours at a temperature of between about 10° and 50°C, preferably 25°C in a solvent such as dichloromethane or DMF, preferably dichloromethane. The coupling of the carboxyl group to the N-methyl-Ser(OBzl) attached to the peptide resin requires catalysis by 4-dimethylaminopyridine (DMAP), in addition to the carbodiimide reagent.

The coupling of successive protected amino acids can be carried out in an automatic polypeptide synthesizer as is well known in the art. The removal of the alpha-N-protecting groups may be performed in the presence of, for example, a solution of trifluoroacetic acid in methylene chloride, hydrogen chloride in dioxane, hydrogen chloride in acetic acid, or other strong acid solution, preferably 50% trifluoroacetic acid in dichloromethane at about ambient temperature. Each protected amino acid is preferably introduced in 0.4M concentration and approximately 3.5 molar excess and the coupling may be carried out in dichloromethane, dichloromethane/DMF mixtures, DMF and the like, especially in methylene chloride at about ambient temperature. The coupling agent is normally DCC in dichloromethane but may be N,N'-di-isopropylcarbodiimide (DIC) or other carbodiimide either alone or in the presence of HOBT, N-hydroxysuccinimide, other N-hydroxyimides or oximes. Alternately, protected amino acid active ester (e.g. p-nitrophenyl, pentafluorophenyl and the like) or symmetrical anhydrides may be used.

At the end of the solid phase synthesis the fully protected polypeptide is removed from the resin. When the linkage to the resin support is of the benzyl ester type, cleavage is by means of aminolysis with an alkylamine or fluoroalkylamine for peptides with a proline C-terminus, or by aminolysis with, for example, ammonia/methanol or ammonia/ethanol for peptides with a glycine C-terminus at a temperature between about 10° and 50°C, preferably about 25°C, for between about 12 and 48 hours preferably about 18 hours. Alternatively, the peptide may be removed from the resin by transesterification, e.g., with methanol, followed by aminolysis or by direct transamidation. The protected peptide may be purified at this point by silica gel chromatography or taken to the next step directly. The removal of the side chain protecting groups from the polypeptide is performed by treating the aminolysis product with, for example, anhydrous liquid hydrogen fluoride in the presence of anisole and dimethylphosphite or other carbonium scavenger, treatment with hydrogen fluoride/pyridine complex, treatment with tris(trifluoroacetyl)boron and trifluoroacetic acid, by reduction with hydrogen and palladium on carbon on polyvinylpyrrolidone, or by reduction with sodium in liquid ammonia. Side chain protecting groups are preferrably removed with liquid hydrogen fluoride in the presence of anisole and dimethylphosphite at a temperature between about -10 and +10°C, preferably about 0°C, for between about 15 minutes and 1 hour. The fully deprotected polypeptide is then purified by a sequence of chromatographic steps employing any or all of the following types: ion exchange on a weakly basic resin in the acetate form; hydrophobic adsorption chromatography on underivatized polystyrene-divinylbenzene (for example Amberlite XAD); silica gel adsorption chromatography; ion exchange chromatography on carboxymethylcellulose; partition chromatography, e.g., on Sephadex G-25, LH-20, or countercurrent distribution; high performance liquid chromatography (HPLC), especially reverse phase HPLC on octyl- or octadecylsilyl-silica bonded phase column packing.

If a racemic amino acid is used in the 6 position, the diastereomeric decapeptide final products are separated, and the desired peptide containing a D-amino acid in the appropriate position is isolated and purified, preferably during the above-described chromatographic process.

The details for the preparation of peptides using classical peptide solution synthesis are described in Example 2.

The following examples are reference examples which will serve to further illustrate the preparation of peptides.

Preparation A

N-(t-Butoxycarbonyl)-N-Methyl-O-Benzyl-L-Serine Cyclohexylamine Salt

Methyl iodide (227.2 g) was added to a solution of N-Boc-O-benzyl-L-serine (23.68 g) in dry and freshly distilled dimethoxyethane (DME) (370 ml) stirred under nitrogen and cooled to 0°C. Subsequently, sodium hydride (50% oil dispersion) (6.4 g) was added in portions over 15 minutes. The reaction mixture was further stirred at 0-5°C for 22 hours and then decomposed by water, and the organic layer was concentrated. The residue was taken up in water (500 ml) and washed with ether (3x100 ml). The aqueous layer was acidified with cold 1N HCl to pH 3.0, and the oil that separated was extracted into ether (3 X 300 ml). The ethereal layer was washed with cold 1N sodium thiosulfate solution (2 x 150 ml) and sodium chloride solution (2 x 150 ml), dried ($Na_2SO_4$) and concentrated. The crude product was dissolved in ether (300 ml), and cyclohexylamine (8.3 g) was added. The salt that separated was filtered and dried to give N-(t-butoxycarbonyl)-N-methyl-O-benzyl-L-serine cyclohexylamine salt (CHA), m.p. 134-136°C. $[\text{alpha}]_D^{24}$ -8.6(C 1,EtOH); Anal. for $C_{22}H_{34}N_2O_5$, Calcd: C, 65.00; H, 8.43; N, 6.89; Found: C, 65.05; H, 8.88; N, 6.91.

Example 1

(pyro)Glu-His-Trp-N-Me-Ser-Tyr-D-Leu-Leu-Arg-Pro-NHEt

In the reaction vessel of a Biosearch 9500 Peptide Synthesizer was placed 1.5 g (1.05 mmol) of Boc-Pro-O-Resin (Merrifield resin). Amino acids were added sequentially to this resin according to the following synthetic cycle:

1. Deblocking, to remove the t-Boc group from the alpha-amino function of the peptide, is carried out using a solution of 45% trifluoroacetic acid (TFA), 2.5% anisole, 2.0% dimethyl phosphite, and 50.5% methylene chloride. The resin is prewashed with the deblocking solution previously described for one minute and then the deblocking reacation is run for 20 minutes.

2. Base wash, to remove and neutralize the TFA used for deprotection, is carried out using a solution of 10% N, N'-diisopropylethylamine in methylene chloride. The resin is washed with base three times for one minute each time after each deblocking step.

3. Coupling reacton is carried out using a 3.5-fold molar excess of 0.4M DMF solution of a t-Boc protected amino acid derivative along with a 3.5-fold molar excess of 0.4M methylene chloride solution of diisopropylcarbodiimide as activator. The activated amino acid is then coupled to the free alpha amino group of the peptide-resin. The reaction time is as described in the following protocol.

4. Wash, each reaction step is followed by three washes of one minute each: one of methylene chloride, one of (1:1) methylene-chloride-DMF, and one of DMF.

Protocol:

The amino acids are coupled to the resin in the following order using the conditions indicated:

| Amino Acid | Wash | Coupling | Deprotection |
|---|---|---|---|
| Boc-Arg(Tos) | basewash | two-1 hr | deblock |
| Boc-Leu | basewash | two-1 hr | deblock |
| Boc-D-Leu | basewash | two-1 hr | deblock |
| Boc-Tyr-(o-Br-Cbz) | basewash | two-1 hr | deblock |
| Boc-N-Me-Ser(OBzl) | basewash | two-1 hr | deblock |
| Boc-N-Formyl-Trp containing 0.1% DMAP | basewash | four-1 hr | deblock |
| Boc-N-im-CBZ-His | basewash | four-1 hr | deblock |
| Cbz-p-Glu | basewash | four-1 hr | none |

Upon the completion of the synthesis the resin is removed from the reaction vessel and dried in vacuo to give the protected polypeptide resin. The protected peptide is removed from the resin upon treatment at room temperature with anhydrous ethylamine with or without 10% DMF or methanol for 48 hours. The resin beads are filtered and washed with methanol. The filtrate is concentrated in vacuo and the residue is triturated with water to give, after filtration and drying, the protected peptide as a white powder. The protecting groups are finally removed upon treatment at 0°C for 1 hour with 5 to 10 ml anhydrous liquid HF in the presence of 1 ml of anisole and 0.5 ml of dimethyl phosphite. The HF is evaporated and the residue is dissolved in methanol and concentrated in vacuo. The residue is washed twice with ether and then dissolved in a solution of (1:1:0.1) water:acetonitrile:acetic acid, filtered, and lyophilized to give 0.7 g of the crude product. The crude peptide is purified by high performance liquid chromatography on a 25 cm x 2.5 cm Dynamax C-18 column (25-40 micron ($\mu$m)) using solvent mixtures in a gradient ranging from 89% $H_2O$/11% $CH_3CN$/

0.1% TFA to 49% H$_2$O/51% CH$_3$CN/0.1% TEA over a period of 50 min, and afterwards changing to 100% CH$_3$CN/ 0.1% TFA over a period of 10 min. The flow rate is 15 ml/min and UV detection is at 260 nM. The product is eluted at 33.7 min as a single peak, collected and lyophilized to give pure (pyro)Glu-His-Trp-N-Me-Ser-Tyr-D-Leu-Leu-Arg-Pro-NHEt as the trifluoroacetate salt. Fab (fast atom bombardment) Mass spec. m/e 1296 (M+H)$^+$. Amino Acid Anal.: 0.8 Pro; 0.8 Arg; 1.0 Leu; 1.0 Tyr; 1.6 Trp; 1.0 His; 1.0 Glu.

Example 2

(pyro)Glu-His-Trp-N-Me-Ser-Tyr-D-Leu-Leu-Arg-Pro-NHEt

(pyro)Glu-His-Trp-N-Me-Ser-Tyr-D-Leu-Leu-Arg-Pro-NHEt was prepared using solution synthesis according to the following scheme:

8

FMOC-L-Ser(O-Bzl)

FMOC-N-Me-L-Ser(O-Bzl)
(a)
                                          Tyr-D-Leu-OEt

FMOC-N-Me-Ser(O-Bzl)-Tyr-D-Leu-OEt
(b)

N-Me-Ser(O-Bzl)-Tyr-D-Leu-OEt

FMOC-Trp                          (c)

FMOC-Trp-N-Me-Ser(O-Bzl)-Tyr-D-Leu-OEt
(d)

Trp-N-Me-Ser(O-Bzl)-Tyr-D-Leu-OEt          Cbz-(pyro)Glu-His
(e)

Cbz-(pyro)Glu-His-Trp-N-Me-Ser(O-Bzl)-Tyr-D-Leu-OEt
(f)

(pyro)Glu-His-Trp-N-Me-Ser-Tyr-D-Leu-OEt
(g)

```
(pyro)Glu-His-Trp-N-Me-Ser-Tyr-D-Leu-OH

  Leu-Arg-Pro-NHEt


                                    (h·)



(pyro)Glu-His-Trp-N-Me-Ser-Tyr-D-Leu-Leu-Arg-Pro-NHEt

                        (i)
```

Details of the synthesis are as follows:

(a) FMOC-N-Me-Ser(O-Bzl)

A suspension of FMOC-Ser(O-Bzl) (4.16 g), paraformaldahyde (2.0 g), and p-toluenesulfonic acid (0.2 g) in toluene (400 ml) was heated under reflux with azeotropic water removal for 45 min. The solution was cooled, diluted with ethyl acetate (250 ml) and washed three times with 5% aqueous $NaHCO_3$, dried ($Na_2SO_4$), and concentrated in vacuo. The residue was purified by silica gel column chromatography eluting with (8:2) hexane:ethyl acetate to give FMOC-Ser(O-Bzl)-oxazolidin-4-one as a crystalline product, m.p. 108-109°C. Fab Mass spec. m/e 430(M+H)+.

FMOC-Ser-(O-Bzl)-oxazolidin-4-one (3.14 g) was dissolved in chloroform (40 ml) and trifluoroacetic acid (40 ml) and triethylsilane (2.55 g) was added. The solution was stirred at room temperature for 22 hours, then concentrated in vacuo. The residue was purified by silica gel column chromatography eluting with (95:5) methylene chloride: methanol to give FMOC-N-Me-Ser(O-Bzl)-OH as a colorless oil. Fab Mass spec., m/e 432 (M+H)+.

(b) FMOC-N-Me-Sec(O-Bzl)-Tyr-D-Leu-OEt

To a stirred solution of Tyr-D-Leu-OEt hydrochloride (1.649 g) in DMF (10 ml) cooled to 0°C was added N-ethylmorpholine (0.59 ml) in DMF (1 ml), followed by a solution of FMOC-N-Me-Ser(O-Bzl)-OH (2.18 g) in DMF (5 ml), followed by a solution of HOBt (0.9315 g) in DMF (5 ml), and followed by a solution of DCC (0.947 g) in DMF (2 ml). The reaction solution was stirred at 0°C for 1 hour and then at room temperature for 4 hours. The solvent was removed in vacuo and the residue was purified by silica gel column chromatography eluting with (95:5) methylene chloride:methanol. The product was obtained as a semisolid. Rf 0.35. Fab Mass spec. m/e 736 (M++H).

(c) N-Me-Ser(O-Bzl)-Tyr-D-Leu-OEt

A solution of FMOC-N-Me-Ser(O-Bzl)-Tyr-D-Leu-OEt (1.95 g) and N,N-diisopropylamine (10 ml) in dry and degassed DMF (10 ml) was stirred at room temperature for 2 hours. The solvent and excess reagents were removed in vacuo and the residue was purified by silica gel column chromatography eluting with (95:5) methylene chloride: methanol. The product was obtained as a low melting solid. Rf 0.24. Fab Mass spec., m/e 514 (M+H)+. Anal for $C_{28}H_{39}N_3O_6$, Calcd: C, 65.47; H, 7.65; N, 8.18; Found: C, 65.10; H, 7.77; N, 7.98.

(d) FMOC-Trp-N-Me-Ser(O-Bzl)-Tyr-D-Leu-OEt

To a stirred solution of N-Me-Ser(O-Bzl)-Tyr-D-Leu-OEt (1.316 g), FMOC-L-Trp (1.09 g) and benzotriazol-1-yloxytris-(dimethylamino) phosphonium hexafluorophosphate (BOP) (1.13 g) in acetonitrile (50 ml) was added triethylamine (0.347 ml). The solution was stirred at room temperature for 5 hours. The solvent was removed in vacuo. The residue was dissolved in ethyl acetate, washed with 5% aqueous $NaHCO_3$, then with IN HCl, and finally with saturated aqueous NaCl solution, dried ($Na_2SO_4$) and concentrated in vacuo. The residue was purified by silica gel column chromatography eluting with (95:5) methylene chloride:methanol. The product was obtained as a semisolid residue. Rf 0.25. Fab Mass spec., m/e 922 (M++H).

(e) Trp-N-Me-Ser(O-Bzl)-Tyr-D-Leu-OEt

A solution of FMOC-Trp-N-Me-Ser(O-Bzl)-Tyr-D-Leu-Et (0.280 g) in acetonitrile (5 ml) and diethylamine (5 ml) was stirred at room temperature for 1 hour. The solvent and excess reagents were removed in vacuo to give the product as a foamy residue. Fab Mass spec. m/e 700 (M+1)+. The product was used in the next step without further purification.

(f) <u>Cbz-(pyro)Glu-His-Trp-N-Me-Ser(O-Bzl)-Tyr-D-Leu-OEt</u>

To a solution of Trp-N-Me-Ser(O-Bzl)-Tyr-D-Leu-OEt (0.2665 g) in DMF (5 ml) cooled to 0°C were added sequentially Cbz-(pyro)Glu-His (0.167 g) in DMF (10 ml), HOBt (0.077 g) in DMF (2 ml), and DCC (0.078 g) in DMF (2 ml). The solution was stirred at 0°C for 2 hours and then at room temperature overnight. The solvent was removed <u>in vacuo</u> and the residue was purified on a silica gel column eluting with (9:1) methylene chloride:methanol. The product was obtained as a solid. Rf 0.317. Fab Mass spec. m/e 1082 $(M+H)^+$.

(g) <u>(Pyro)Glu-His-Trp-N-Me Ser-Tyr-D-Leu-OEt</u>

A solution of Cbz-(pyro)Glu-His-Trp-N-Me-Ser(O-Bzl)-Tyr-D-Leu-OEt (0.787 g) in (9:1) DMF-water (15 ml) was hydrogenated overnight under 4 atm. pressure and in the presence of 10% $Pd(OH)_2$/C (0.79 g). The catalyst was filtered and the filtrate was concentrated <u>in vacuo.</u> The residue was triturated with water to give the desired product as an amorphous solid. Fab Mass spec. m/e 857 $(M+H)^+$.

(h) <u>(pyro)Glu-His-Trp-N-Me-Ser-Tyr-D-Leu-OH</u>

To a solution of (pyro)Glu-His-Trp-N-Me-Ser-Tyr-D-Leu-OEt (0.519 g) in (1:1) dioxane-water (16 ml) cooled to 0°C was added 2N aqueous NaOH (0.6 ml). The resulting solution was stirred at 0°C for 4 hours, then acidified with 0.1M aqueous HCl to pH 5.0 and lyophilized. Fab Mass spec. of the crude product showed m/e 830 for $(M^+H)$. The crude product was taken to the next step without any additional purification.

(i) <u>(pyro)Glu-His-Trp-N-Me-Ser-Tyr-D-Leu-Leu-Arg-Pro-NHEt</u>

To a solution of Leu-Arg-Pro-NHEt dihydrochloride (0.159 g) in DMF (2 ml) cooled to 0°C was added N-ethylmorpholine (0.042 ml) in DMF (0.2 ml), followed by sequential additions of (pyro)Glu-His-Trg-N-Me-Ser-Tyr-D-Leu-OH (0.3 g) in DMF (5 ml), HOBt (0.066 g) in DMF (2 ml), and DCC (0.0677 g) in DMF (2 ml). The resulting solution was stirred at 0°C for 2 hours and then at room temperature overnight. The solvent was removed <u>in vacuo.</u> The residue was dissolved in (1:9) acetic acid-water and the insoluble material was filtered. The filtrate was lyophilized. The powder obtained was purified by high performance liquid chromatography (HPLC) using a 25 cm x 2.5 cm Dynamax C-18 column (25-40 micron (μm)) and solvent mixture gradients ranging from 90% $H_2O$/11% $CH_3CN$/ 0.1% TFA to 49% $H_2O$/51% $CH_3CN$/0.1% TFA over a period of 50 mins. The flow rate was 15 ml/min and UV detection was at 260 nM. The product was eluted at 30.4 min, was collected and lyophilized to give pure (pyro) Glu-His-Trp-N-Me-Ser-Tyr-D-Leu-Arg-Pro-NHEt as the trifluoroacetate salt. Fab Mass spec. m/e 1223 $(M+H)^+$. Amino Acid Anal.: 0.8 Pro; 0.8 Arg; 1.8 Leu; 1.0 Tyr; 1.0 His; 1.0 Glu.

<u>Example 3</u>

<u>N-Ac-D-2-Nal-N-Me-D-4-Cl-Phe-D-3-Pal-Ser-Lys(epsilon-N-nic otinoyl)-D-Lys(N-epsilon-nicotinoyl)-Leu-Lys(N-epsilon-isopropyl)-Pro-D-AlaNH$_2$</u>

Using a procedure and a synthetic protocol similar to those described in Example 1 but substituting BOC-D-Ala-NH-Resin (4-methyl-benzyhydrylamine resin) for BOC-Pro-O-Resin (Merrifield resin) and coupling the amino acids according to the following order and coupling protocol:

| # | Amino Acid | Coupling |
|---|---|---|
| 1 | BOC-Pro | two-1h |
| 2. | BOC-Lys(N-epsilon-isopropyl-N-epsilon-CBZ) | two-1h |
| 3. | BOC-Leu | two-1h |
| 4. | BOC-D-Lys(N-epsilon-FMOC) | two-1h |
| 5. | BOC-Lys(N-epsilon-FMOC) | two-1h |
| 6. | BOC-3-D-Pal | four-2h |
| 7. | BOC-N-Me-D-4-Cl-Phe | four-1h |
| 8. | N-Ac-D-2-Nal containing 0.1% DMAP | four-1h |

Upon completion of the synthesis the resin is treated with 20% piperidine in $CH_2Cl_2$ solution for 8 hours to remove the FMOC protecting groups from the two Lys. After several washes with $CH_2Cl_2$ and drying <u>in vacuo</u>, the peptide on the resin is coupled with nicotinic acid using the peptide synthesizer and the two-lh coupling protocol. Subsequently the peptide is cleaved from the resin with HF at 0°C for lh in the presence of anisole and dimethylphosphite to give N-Ac-D-2-Nal-N-Me-D-4-Cl-Phe-3-Pal-Ser-Lys-(N-epsilon-nicotinoyl)-D-Lys-(N-epsilon-nicotinoyl)-Leu-Lys(N-epsilon-

EP 0 400 065 B1

isopropyl)-Pro-D-AlaNH$_2$ as a crude product. The peptide can be purified by HPLC using the conditions previously described.

<u>Example 4</u>

<u>N-Ac-D-2-Nal-D-4-Cl-Phe-D-3-Pal-Ser-N-Me-Tyr-D-Lys-(N-epsilon-picoloyl)-Leu-Lys-(N-epsilon-isopropyl)-Pro-D AlaNX$_2$</u>

Using the same procedure, protocol and amino acids as described in Example 3, but substituting BOc-N-Me-Tyr (O-2,6-diCl-Bzl) for BOC-Lys-(N-epsilon-FMOC), adding 0.1% DMAP only to the DMF solution of BOC-Ser(OBzl), and at the end coupling with picolinic acid instead of nicotinic acid. Following workup and HPLC purification, the desired compound can be obtained.

<u>Assay Procedures</u>

The biological activities of the compound of the invention are determined by the following assays:

(a) <u>Receptor Binding.</u> A radioligand receptor binding assay is performed in a similar way to that described in the literature (J. Marion et al., Mol. Pharmacol. <u>19</u> 399 (1981)). [D-Leu$^6$-des Gly$^{10}$]-LHRH ethyl amide was radioiodinated by the chloramine-T method and used as the radioligand. Pituitary membranes containing LHRH receptors are prepared in batches from quick-frozen rat pituitaries obtained from Hilltop Labs. The radioligand (50pM), receptors, and compounds to be tested are coincubated for 2 hours at 4°C. Bound ligand is separated from free ligand via centrifugation and aspiration. Compounds are tested at six half-log concentration increments, and the negative log of the equilibrium dissociation constant ($pK_I$) is calculated from the concentration which displaces 50% of specifically bound radioligand.

(b) <u>In vitro LH Release.</u> This assay has been adopted from the literature (H.A. Jinnah and P.M. Conn, Endrocrinology <u>118</u> 2599 (1986)). Rat pituitaries are removed from immature female rats, minced, and dissociated with collagenase/hyaluronidase. They are allowed to attach to 48-well microtiter plates for 48-72 hours, then are exposed to test compounds for 3 hours at 37°C. The medium is assayed for released LH by RIA (radioimmunoassay).

For assaying LHRH antagonists, exogenous superagonist [D-Leu$^6$-Pro$^9$NHEt]LHRH is added. The suppression of LH release by the antagonist is dose related. The assay determines the potencies of the LHRH antagonists from the negative log of the concentration which produces half-maximum suppression of LH ($pA_2$).

(c) <u>In vivo LH Release.</u> The compound to be tested is administered to castrated rats intraveneously and the serum LH concentration at various time points is measured by RIA. The time integrated LH response is calculated and the dose producing half-maximal LH release ($ED_{50}$) is reported.

(d) <u>Stability against enzymatic degradation.</u> The intestinal stability of the compound of the invention was determined using <u>in vitro</u> rat jejunum in a reperfusion system. The fractional mucosal loss was an indicator of the relative rate of degradation of the compounds thirty minutes after introduction of the luminal bath. See Figure 1.

**Claims**

**Claims for the following Contracting States : BE, CH, LI, DE, FR, GB, IT, NL, SE**

1. N-Ac-D-2-Nal-D-4-Cl-Phe-D-3-Pal-Ser-N-Me-Tyr-D-Lys(N-epsilon-nicotinoyl)-Leu-Lys(N-epsilon-isopropyl)-Pro-D-AlaNH$_2$; or a pharmaceutically acceptable salt thereof.

2. A pharmaceutical composition for suppressing levels of sex hormones in male or female mammals, comprising a pharmaceutical carrier and a therapeutically effective amount of an LHRH antagonist compound of Claim 1.

3. Use of an LHRH antagonist compound of Claim 1 for preparing a medicament for suppressing levels of sex hormones in male or female mammals in need of such treatment.

4. A process for the preparation of a compound of Claim 1 comprising sequentially coupling suitably protected amino acids or amino acid analogs, followed by removal of the protecting groups.

5. The process of Claim 4 wherein sequences of two or more suitably protected amino acids or amino acid analogs are coupled and the resulting peptides are coupled, followed by removal of the protecting groups.

### Claims for the following Contracting State : GR

1. A process for the preparation of the compound N-Ac-D-2-Nal-D-4-Cl-Phe-D-3-Pal-Ser-N-Me-Tyr-D-Lys(N-epsilon-nicotinoyl)-Leu-Lys(N-epsilon-isopropyl)-Pro-D-AlaNH$_2$ or a pharmaceutically acceptable salt thereof, said process comprising sequentially coupling suitably protected amino acids or amino acid analogs, followed by removal of the protecting groups.

2. The process of Claim 1 wherein sequences of two or more suitably protected amino acids or amino acid analogs are coupled and the resulting peptides are coupled, followed by removal of the protecting groups.

3. Use of the LHRH antagonist compound N-Ac-D-2-Nal-D-4-Cl-Phe-D-3-Pal-Ser-N-Me-Tyr-D-Lys(N-epsilon-nicotinoyl)-Leu-Lys(N-epsilon-isopropyl)-Pro-D-AlaNH$_2$ or a pharmaceutically acceptable salt thereof for preparing a medicament for suppressing levels of sex hormones in male or female mammals in need of such treatment.

### Claims for the following Contracting State : ES

1. A process for the preparation of the compound N-Ac-D-2-Nal-D-4-Cl-Phe-D-3-Pal-Ser-N-Me-Tyr-D-Lys(N-epsilon-nicotinoyl)-Leu-Lys(N-epsilon-isopropyl)-Pro-D-AlaNH$_2$ or a pharmaceutically acceptable salt thereof, said process comprising sequentially coupling suitably protected amino acids or amino acid analogs, followed by removal of the protecting groups.

2. The process of Claim 1 wherein sequences of two or more suitably protected amino acids or amino acid analogs are coupled and the resulting peptides are coupled, followed by removal of the protecting groups.

### Patentansprüche

### Patentansprüche für folgende Vertragsstaaten : BE, CH, LI, DE, FR, GB, IT, NL, SE

1. N-Ac-D-2-Nal-D-4-Cl-Phe-D-3-Pal-Ser-N-Me-Tyr-D-Lys(N-epsilon-nicotinoyl)-Leu-Lys(N-epsilon-isopropyl)-Pro-D-AlaNH$_2$; oder ein pharmazeutisch verträgliches Salz davon.

2. Pharmazeutische Zubereitung zur Unterdrückung der Sexualhormonpegel bei männlichen oder weiblichen Säugern, die einen pharmazeutischen Träger und eine therapeutisch wirksame Menge einer LHRH Antagonistenverbindung nach Anspruch 1 umfaßt.

3. Verwendung einer LHRH Antagonistenverbindung nach Anspruch 1 zur Herstellung eines Medikamentes zur Unterdrückung der Sexualhormonpegel bei männlichen oder weiblichen Säugern, die einer solchen Behandlung bedürfen.

4. Verfahren zur Herstellung einer Verbindung nach Anspruch 1, das die sequentielle Kupplung geeignet geschützter Aminosäuren oder Aminosäurenanaloga, gefolgt von der Entfernung der Schutzgruppen, umfaßt.

5. Verfahren nach Anspruch 4, wobei Sequenzen von zwei oder mehr geeignet geschützten Aminosäuren oder Aminosäureanaloga gekuppelt werden und wobei die resultierenden Peptide gekuppelt werden, gefolgt von der Entfernung der Schutzgruppen.

**Patentansprüche für folgenden Vertragsstaat : ES**

1. Verfahren für die Herstellung der Verbindung N-Ac-D-2-Nal-D-4-Cl-Phe-D-3-Pal-Ser-N-Me-Tyr-D-Lys(N-epsilon-nicotinoyl)-Leu-Lys(N-epsilon-isopropyl)-Pro-D-AlaNH$_2$ oder eines pharmazeutisch verträglichen Salzes davon, wobei das Verfahren die sequentielle Kupplung geeignet geschützter Aminosäuren oder Aminosäurenanaloga, gefolgt von der Entfernung der Schutzgruppen, umfaßt.

2. Verfahren nach Anspruch 1, wobei Sequenzen von zwei oder mehr geeignet geschützten Aminosäuren oder Aminosäureanaloga gekuppelt werden und wobei die resultierenden Peptide gekuppelt werden, gefolgt von der Entfernung der Schutzgruppen.

**Patentansprüche für folgenden Vertragsstaat : GR**

1. Verfahren für die Herstellung der Verbindung N-Ac-D-2-Nal-D-4-Cl-Phe-D-3-Pal-Ser-N-Me-Tyr-D-Lys(N-epsilon-nicotinoyl)-Leu-Lys(N-epsilon-isopropyl)-Pro-D-AlaNH$_2$ oder eines pharmazeutisch verträglichen Salzes davon, wobei das Verfahren die sequentielle Kupplung geeignet geschützter Aminosäuren oder Aminosäurenanaloga, gefolgt von der Entfernung der Schutzgruppen, umfaßt.

2. Verfahren nach Anspruch 1, wobei Sequenzen von zwei oder mehr geeignet geschützten Aminosäuren oder Aminosäureanaloga gekuppelt werden und wobei die resultierenden Peptide gekuppelt werden, gefolgt von der Entfernung der Schutzgruppen.

3. Verwendung der LHRH Antagonistenverbindung N-Ac-D-2-Nal-D-4-Cl-Phe-D-3-Pal-Ser-N-Me-Tyr-D-Lys(y-epsilon-nicotinoyl)-Leu-Lys(N-epsilon-isopropyl)-Pro-D-AlaNH$_2$ oder eines pharmazeutisch verträglichen Salzes davon zur Herstellung eines Medikamentes zur Unterdrückung der Sexualhormonpegel bei männlichen oder weiblichen Säugetieren, die einer solchen Behandlung bedürfen.

**Revendications**

**Revendications pour les Etats contractants suivants : BE, CH, LI, DE, FR, GB, IT, NL, SE**

1. N-Ac-D-2-Nal-D-4-Cl-Phe-D-3-Pal-Ser-N-Me-Tyr-D-Lys(N-epsilon-nicotinoyl)-Leu-Lys(N-epsilon-isopropyl)-Pro-D-AlaNH$_2$; ou un sel acceptable pharmaceutiquement de celui-ci.

2. Composition pharmaceutique pour la suppression de niveaux d'hormones sexuelles chez les mammifères mâles ou femelles, comprenant un support pharmaceutique et une quantité efficace thérapeutiquement d'un composé antagoniste LHRH selon la revendication 1.

3. Utilisation d'un composé antagoniste LHRH selon la revendication 1 pour la préparation d'un médicament pour la suppression de niveaux d'hormones sexuelles chez les mammifères mâles ou femelles nécessitant un tel traitement.

4. Procédé de préparation d'un composé selon la revendication 1 comprenant le couplage par séquences d'acides aminés ou d'analogues d'acides aminés convenablement protégés, suivi par l'enlèvement des groupes protecteurs.

5. Procédé selon la revendication 4, caractérisé en ce que des séquences de deux ou plus d'acides aminés convenables sont couplés et que les peptides résultants sont couplés, suivi par l'enlèvement des groupes protecteurs.

**Revendications pour l'Etat contractant suivant : ES**

1. Procédé de préparation du composé composé N-Ac-D-2-Nal-D-4-Cl-Phe-D-3-Pal-Ser-N-Me-Tyr-D-Lys(N-epsilon-nicotinoyl)-Leu-Lys(N-epsilon-isopropyl)-Pro-D-AlaNH$_2$; ou un sel acceptable pharmaceutiquement de celui-ci, ledit procédé comprenant le couplage par séquences d'acides aminés ou d'analogues d'acides aminés convenablement protégés, suivi par l'enlèvement des groupes protecteurs.

2. Procédé selon la revendication 1, caractérisé en ce que des séquences de deux ou plus d'acides aminés ou d'analogues d'acides aminés convenablement protégés sont couplées et que les peptides résultants sont couplés, suivi par l'enlèvement des groupes protecteurs.

**Revendications pour l'Etat contractant suivant : GR**

1. Procédé de préparation du composé N-Ac-D-2-Nal-D-4-Cl-Phe-D-3-Pal-Ser-N-Me-Tyr-D-Lys(N-epsilon-nicotinoyl)-Leu-Lys( N-epsilon-isopropyl)-Pro-D-AlaNH$_2$; ou un sel acceptable pharmaceutiquement de celui-ci, ledit procédé comprenant le couplage par séquence d'acides aminés ou d'analogues d'acides aminés convenablement protégés, suivi par l'enlèvement des groupes protecteurs.

2. Procédé selon la revendication 1, caractérisé en ce que des séquences de deux ou plus d'acides aminés ou d'analogues d'acides aminés convenablement protégés sont couplées et que les peptides résultants sont couplés, suivi par l'enlèvement des groupes protecteurs.

3. Utilisation du composé antagoniste LHRH N-Ac-D-2-Nal-D-4-Cl-Phe-D-3-Pal-Ser-N-Me-Tyr-D-Lys(N-epsilon-nicotinoyl)-Leu-Lys( N-epsilon-isopropyl)-Pro-D-AlaNH$_2$; ou un sel acceptable pharmaceutiquement de celui-ci, pour la préparation d'un médicament pour la suppression de niveaux d'hormones sexuelles chez les mammifères mâles ou femelles nécessitant un tel traitement.